# EUROPÄISCHE PATENTSCHRIFT

(11) **EP 1 194 524 B1**
(45) Veröffentlichungstag und Bekanntmachung des Hinweises auf die Patenterteilung: **19.11.2003**
(21) Anmeldenummer: 00947935.3
(22) Anmeldetag: 05.07.2000
(51) Int. Cl.: C12M 3/06

(54) **BIOREAKTOR**
BIOREACTOR
BIOREACTEUR

(30) Priorität: 12.07.1999 DE 19932439
(43) Veröffentlichungstag der Anmeldung: 10.04.2002
(73) Patentinhaber: Sefar AG, 8803 Rüschlikon (CH)
(72) Erfinder: WECHSLER, Thomas, D-8004 Zürich (CH); BÄR, Ulrich, CH-9410 Heiden (CH); OEHR, Christian, D-71083 Herrenberg (DE); GRAEVE, Thomas, D-70372 Stuttgart (DE)
(74) Vertreter: Wunderlich, Rainer, Dipl.-Ing.
(86) Internationale Anmeldenummer: EP0006355
(87) Internationale Veröffentlichungsnummer: WO01004262

(56) Entgegenhaltungen:
- EP-A- 0 113 328
- EP-A- 0 909 811
- US-A- 4 833 083
- US-A- 5 605 835

## Beschreibung

Die Erfindung betrifft einen Bioreaktor sowie ein Verfahren zur Kultivierung organischen Materials gemäß dem Oberbegriff der Ansprüche 1 bzw. 10.

Die Kultivierung organischen Materials, vor allem menschlicher oder tierischer Zellen gewinnt in der medizinischen Diagnostik, Therapie und Pharmakologie zunehmend an Bedeutung.

Von besonderem Interesse ist hierbei die Vermehrung von hämatopoetischen Stammzellen. Diese werden einem Patienten vor einer Strahlen- oder Chemotherapie entnommen und sollen in möglichst großer Anzahl dem Patienten nach Abschluß der Strahlen- und Chemotherapie retransplantiert werden.

Bei diesem Verfahren ist unter anderem die Kryokonservierung gebräuchlich, bei welcher entnommene Blutstammzellen-während der Dauer der Strahlen- oder Chemotherapie eingefroren werden. Allerdings ist hierdurch keine Anreicherung der Zellen möglich. Vielmehr wird die Anzahl lebender Zellen sowie deren Vitalität deutlich reduziert.

Es wurden bereits Methoden zur Kultivierung und Anreicherung verschiedener Zellen menschlichen Ursprungs entwickelt und etabliert.

Typischerweise werden Zellen in Behältern oder Petrischalen kultiviert, in welchen sich ein für die Kultivierung des jeweiligen Zelltyps geeignetes Nährmedium befindet. Während der Kultivierung sind im allgemeinen mehrere Behandlungsschritte notwendig, wie beispielsweise der Austausch des Nährmediums sowie ein Umsetzen kultivierter Zellen in andere Behältnisse.

Durch das erforderliche mehrfache Eingreifen in den Kultivierungsprozess wächst die Gefahr der Kontamination des Zellmaterials, beispielsweise durch Laborgeräte oder Umgebungsluft, wodurch das zu kultivierende Material für die weitere Verwendung unbrauchbar wird.

Die Handhabung des gesamten Kultivierungsprozesses gestaltet sich insgesamt relativ aufwendig, so daß ein klinischer Einsatz im großen Maßstab kaum durchführbar ist.

Weiter wurde eine Kultivierung von Zellen in Hohlfasern versucht. Die Zellen in den Hohlfasern werden dabei von der Faseraußenseite diffusiv mit Nährstoffen versorgt. Zu Beginn der Zellkultivierung können dabei relativ gute Wachstumsraten erreicht werden, wobei mit zunehmender Anzahl von Zellen in den räumlich begrenzten Hohlfasern eine weitere Vermehrung problematisch wird.

Eine ähnliche Problematik besteht bei dem Bioreaktor und dem Verfahren gemäß der EP 0 121 981 A1. Ein poröser, monolithischer Keramikblock, welcher mit einer Vielzahl von feinen, parallel verlaufenden.Kanälen durchzogen ist, wird mit Zellen beimpft. Die Zellen lagern sich an der porösen Innenseite der Kanäle an, welche von einer Nährflüssigkeit durchströmt werden. Ein weiterer Bioreaktor zur diffusiven Versorgung von Zellen, welcher einen schichtweisen Aufbau aufweist, ist aus der US-A-5,605,835 bekannt.

Aus der gattungsbildenden EP-A-0 909 811 geht ein Bioreaktor und ein Verfahren mit konvektiver Versorgung des organischen Materials hervor. Bei diesem bekannten Bioreaktor. ist ein zylindrischer Aufbau mit konzentrisch angeordneten Trennwänden vorgesehen, zwischen welchen in einem ringförmigen Aufnahmeraum das organische Material angeordnet ist. Abhängig von der Art des zu kultivierenden organischen Materials muss der Aufnahmeraum in Größe und Form angepasst werden.

Ein weiterer Bioreaktor mit einem vergleichbaren zylindrischen Aufbau ist aus der US-A-4,833,083 bekannt.

In der EP-A-0 113 328 ist ein Bioreaktor mit einer zylindrischen Kammer offenbart. Die Zu- und Abführung des Nährmediums erfolgt über eine Vielzahl poröser Rohre, welche in die Kammer hineinragen.

Der Erfindung liegt die **Aufgabe** zugrunde, eine Vorrichtung und ein Verfahren zur Kultivierung organischen Materials zu schaffen, welche einerseits eine intensive Kultivierung eines organischen Materials erlauben und andererseits besonders einfach und zuverlässig handhabbar sind.

Die Aufgabe wird erfindungsgemäß durch den Bioreaktor mit den Merkmalen des Anspruchs 1 sowie mit dem Verfahren gemäß Anspruch 10 gelöst.

Der Bioreaktor basiert auf der konvektiven Versorgung des organischen Materials mit Nährmedium. Diese Versorgung kann kontinuierlich öder quasi-kontinuierlich erfolgen. Hierdurch kann eine nahezu optimale Bereitstellung der notwendigen Nährstoffe über die gesamte Zeitspanne der Kultivierung gewährleistet werden, wobei gleichzeitig durch die Strömung des Nährmediums für das Zellwachstum abträgliche Stoffwechselprodukte rasch entfernt werden. Versuche mit hämopostischen Stammzellen haben hervorragende Wachstums- und Vitalitätsraten erbracht.

Der Bioreaktor umfaßt ein geschlossenes Gehäuse mit mindestens einem Zu- und Ablauf und mindestens einem Strömungskanal, wobei zur Erzeugung der Strömung ein Gefälle oder gängige Pumpeneinrichtungen, etwa Schlauchpumpen, eingesetzt werden können.

Prinzipiell sind aber auch andere Vorrichtungen geeignet, die das Nährmedium in Strömung versetzen können. Die Strömungsgeschwindigkeit und der Durchfluß werden so eingestellt, daß das organische Material in der Aufnahmeeinrichtung weitgehend immobilisiert bleibt. Das Nährmedium strömt quer durch die Aufnahmeeinrichtung von einem Trennwandelement zum anderen, wobei das organische Material im wesentlichen quer zur Strömung angeordnet ist.

Mit dem erfindungsgemäßen Bioreaktor wird eine ununterbrochene Versorgung des organischen Materials mit den erforderlichen Nährmedien und Stoffen durch Zuleitung während des gesamten Kultivierungsprozesses ermöglicht, wodurch sich die Handhabung und Durchführung des Kultivierungsprozesses stark vereinfacht. Durch das Wegfallen eines mehrfachen externen Eingreifens in den Kultivierungsprozess sowie der damit verbundenen höheren Kontaminationsgefahr ist der erfindungsgemäße Reaktor insbesondere auch für den klinischen Einsatz in groBem Umfang geeignet.

Die gleichmäßig gute Versorgung des organischen Materials mit Nährstoffen erlaubt eine intensive Kultivierung des Materials, wobei Anreicherungswerte der Zellen den Faktor 10 und größer erzielen können. Zum Vergleich werden bei der Kultivierung von Zellen in Kulturbehältern oder Petrischalen trotz eines wesentlich größeren Aufwandes typischerweise Anreicherungsfaktoren lediglich zwischen 2 und 4 erreicht.

Ganz allgemein ist der erfindungsgemäße Bioreaktor zur Kultivierung von verschiedenartigstem organischen Material geeignet. Vorzugsweise handelt es sich hierbei um einfache Strukturen, wie Bakterien, Viren, Pilze oder Körperzellen. Hierzu zählen neben den Stammzellen unter anderem mikround makrovaskuläre Endothelzellen aus Milz, Nebenniere und Aorta, unterschiedliche Zelltypen aus der Cornea, Augenlinsen- und Retinazellen, Haut-, Knochen- sowie Knochenmarkzellen. Prinzipiell ist aber auch die Kultivierung komplexer Strukturen, etwa von ganzen Organen oder Teilen davon denkbar.

Der erfindungsgemäße Bioreaktor zeichnet sich dadurch aus, daß die Aufnahmeeinrichtung zumindest zwei Trennwandelemente aufweist, durch welche ein Aufnahmeraum umschlossen ist, daß in dem Aufnahmeraum das organische Material angeordnet ist und daß die Trennwandelemente einerseits durchlässig für das Nährmedium und andererseits im wesentlichen undurchlässig für das organische Material ausgebildet sind. Durch die Undurchlässigkeit der Trennwandelemente für das organische Material wird zum einen eine definierte Immobilisierung des organischen Materials in der Aufnahmeeinrichtung erreicht. Es wird ein Wegspülen des organischen Materials verhindert, da dieses in einem definierten Raum in der Strömung eingeschlossen ist. In diesem definierten Raum, der sich vorzugsweise über den gesamten Strömungsquerschnitt erstreckt und relativ schmal ausgebildet ist, kann sich das organische Material auch in gewissem Umfang bewegen, wodurch sich eine gleichmäßig gute Besiedelung mit Zellen ergibt. Zum anderen ist aufgrund der Durchlässigkeit der Trennwandelemente für das Nährmedium weiter eine gute konvektive Versorgung des organischen Materials und damit eine intensive Kultivierung desselben gewährleistet.

Prinzipiell sind zur Verwendung in den Trennwandelementen verschiedenste Materialien denkbar, die die erforderliche Durchlässigkeit für ein zuzuführendes Medium aufweisen. Sie können z.B. aus Gewebe, Gewirke oder Filzen oder aus anderen permeablen Werkstoffen bestehen. Gewebe haben sich als besonders zweckmäßig für Bioreaktoren zur Kultivierung von Leberzellen erwiesen. Gewebe mit ihren relativ groben Maschen erzeugen eine hervorragende Diffusorwirkung bei dem strömenden Nährmedium.

Es ist vorgesehen, dass die Trennwandelemente eine Membran aufweisen. Da sich Membranen mit unterschiedlichen Eigenschaften hinsichtlich ihrer Durchlässigkeit sowie ihres selektiven Verhaltens herstellen lassen, kann die Versorgung der im Bioreaktor zu kultivierenden Zellen mit bestimmten Stoffen durch den Einsatz einer entsprechend geeigneten Membran gezielt beeinflußt werden. Darüber hinaus ist auch die mechanische Stabilität der Trennwandelemente durch die Wahl von unterschiedlich verstärkten Membranen gezielt einstellbar und an die jeweiligen Erfordernisse des organischen Materials, z.B. bei adhärenten Zellen, anpaßbar. Zur Verstärkung der Membran können beispielsweise textile Verstärkungen, wie Gewebe oder Gewirke, dienen. Es ist ein Einsatz organischer oder anorganischer Membranen, beispielsweise aus einem Polymer, Metall oder Keramik oder einer Kombination aus diesen Materialien möglich.

Für eine besonders gute Immobilisierung des organischen Materials ist bei dem erfindungsgemäßen Bioreaktor vorgesehen, daß die Aufnahmeeinrichtung ein Trägerelement aufweist, welches zum Anlagern des organischen Materials ausgebildet ist und für das Nährmedium durchlässig ist. Ein Anlagern des organischen Materials an das im wesentlichen flächige Trägerelement kann durch eine spezielle Struktur des Trägerelementes und/oder durch den Strömungsdruck erreicht werden. Das Trägerelement kann in Kombination mit den Trennwandelementen die Aufnahmeeinrichtung bilden. Diese Ausführung eignet sich unter anderem für die Kultivierung von Implantaten, etwa von in vitro-gezüchteten Hautflächen, für welche eine großflächige Anordnung der immobilisierten Zellen erforderlich ist.

Hierbei ist es vorteilhaft, wenn das Trägerelement ein textiles Trägermaterial umfaßt. Durch die entsprechende Wahl, z.B. von Gewebeart und -material, Filamentstärke, Maschenweite und Fadenzahl lassen sich in einfacher Weise für je-. den Anwendungsfall ein nahezu ideales Verhältnis zwischen Oberfläche und Reaktorvolumen sowie gute Durchflußeigenschaften für die Nährstoffversorgung der Zellen einstellen. Dies erlaubt eine gezielte Beeinflussung und Förderung der Kultivierung des organischen Materials.

Als textile Trägermaterialien eignen sich hierbei technische Gewebe, Gewirke und Gelege, bei welchen die Struktur aus Monofilamenten oder Drähten exakt definiert wird. Die Monofilamente oder Drähte können beispielsweise aus Metall, Keramik, synthetischen und/oder natürlichen Materialien, wie Zellulose, mit und ohne Oberflächenbeschichtungen bestehen. In bestimmten Fällen sind auch Multifilamentgewebe zweckmäßig, bei welchen die die Gewebestruktur definierenden Fäden ihrerseits aus einer Vielzahl kleinerer Fäden bestehen.

Eine gezielte Einlagerung von Zellen in das Trägerelement kann durch eine dreidimensionale Struktur erreicht werden. Das Trägerelement kann beispielsweise ein sogenanntes dreidimensionales technisches Gewebe sein. Derartige Gewebe weisen zwei oder mehrere übereinanderliegende und zum Teil verbundene oder verwobene Gewebematrizen auf, welche einen sicheren Halt für eingelagertes Zellmaterial bieten. Weiterhin kann eine dreidimensionale Struktur durch Falten, Plissieren oder Rollen eines etwa zweidimensionalen Elements erfolgen. Überdies kann eine Gerüststruktur des Trägerelements oder ein Aufbau aus Strukturelementen, wie zum Beispiel Rohrkörper oder Waben, vorgesehen sein. Schließlich können auch sogenannte non-woven-Materialien und Vliesstoffe zur Anwendung kommen.

Bei der Verwendung von technischen Geweben als Trägerelement ist es von Vorteil, wenn das technische Gewebe oberflächenbehandelt ist und eine bioverträgliche Oberfläche mit einer Struktur für eine Adhäsion des organischen Materials ausgebildet ist. Auf diese Weise läßt sich die Oberfläche eines stabilen Gewebes, etwa aus Polyester, Polyamid, einem Trifluorethylen/Ethylen-Copolymer, Metall oder Keramik, für verschiedene Zwecke gezielt funktionalisieren. So kann das Zellwachstum durch die Erzeugung einer hydrophilen Gewebeoberfläche oder durch die Erhöhung der Konzentration an stickstoffhaltigen funktionellen Gruppen positiv beeinflußt werden. Andere Substanzen, wie z.B. Immunglobulin G (IgG), dagegen werden bevorzugt an hydrophoben Oberflächen adsorbiert.

Eine besonders effektive Oberflächenbehandlung stellen hierbei Niedertemperatur-Plasmaverfahren dar, mit welchen beispielsweise Textilmaterialien aus Polymer, Metall oder Keramik und Membrane gezielt mit einer inerten Oberfläche beschichtet und damit funktionalisiert werden können, ohne daß das zu behandelnde Gewebe aggressiven Lösungen oder hohen Temperaturen ausgesetzt werden muß. Auf diese Weise lassen sich unterschiedliche Materialeigenschaften, wie z.B. eine hohe mechanische Stabilität eines Trägergrundmaterials systematisch mit gewünschten Oberflächeneigenschaften, wie z.B. Hydrophilie und Zelladhäsion, kombinieren.

Es ist vorgesehen, daß die Aufnahmeeinrichtung vorzugsweise kreisrund ausgebildet ist. Bei einem kreisrund ausgebildeten Hohlraum in der Flachzelle wird eine besonders gute Strömung und damit eine gleichmäßige Versorgung der zu kultivierenden Zellen mit Nährmedium gewährleistet. Die Flachzelle kann schichtweise, etwa aus verklebten Kunststoffelementen aufgebaut sein, wodurch sich ein kompakter und zugleich einfach herzustellender Bioreaktor ergibt. Die einfache Herstellbarkeit erlaubt sogar den Einsatz des Bioreaktors als Einmalartikel, was für medizinische Anwendungen vorteilhaft sein kann. Gleichzeitig ist die Flachzelle auch so robust, daß eine Sterilisation durch Autoklavieren oder durch γ-Sterilisieren möglich ist. Für. das Beimpfen und Ernten größeren organischen Materials, etwa von Implantaten, ist der Bioreaktor montierbar bzw. demontierbar.

Bei einer weiteren Ausgestaltung der Erfindung ist es vorteilhaft, wenn mehrere Flachzellen als Module in einer Strömungsrichtung parallel und/oder seriell angeordnet sind. Hierbei kann das aus einer ersten Flachzelle ausströmende Medium unmittelbar, weiteren. Flachzellen zugeführt werden, so daß eine besonders effektive Nutzung des Nährmediums sowie eine eventuelle Verwertung von Stoffwechselprodukten einer vorausgehenden Zelle möglich ist. Zweckmäßig ist in der Regel jedoch eine parallele Anordnung, um eventuelle Vergiftungen durch Stoffwechselprodukte auszuschließen.

In einer bevorzugten Ausführungsform des Bioreaktors ist eine Steuereinrichtung vorgesehen, durch welche die Strömungserzeugungs-Einrichtung, eine Temperatureinstelleinheit, eine Begasungseinheit und/oder weitere Versorgungseinheiten steuerbar und/oder regelbar sind. Hierdurch läßt sich der Kultivierungsprozeß des organischen Materials über verschiedene Parameter, wie DurchfluBgeschwindigkeit, Durchflußmenge, Temperatur und Druck des Nährmediums sowie die Zu- und Abführung weiterer Medien und Stoffe gezielt beeinflussen.

Insbesondere ist durch eine Steuerung bzw. Regelung der unterschiedlichen Einheiten von der Steuereinrichtung durch eine zentrale Vorgabe bestimmter Steuer- bzw. Regelprogramme möglich, die, je nach Anforderung, auf einzelne organische Materialien oder Kultivierungsprozesse abgestimmt sind. Auf diese Weise wird eine bedarfsgerechte Einstellung und Steuerung unterschiedlicher Prozeßparameter ermöglicht.

Hierbei ist es besonders vorteilhaft, wenn in einer Strömungsrichtung nach der Aufnahmeeinrichtung eine Sensoreinrichtung angeordnet ist, durch welche physikalische und chemische Zustandswerte des Nährmediums ermittelbar sind und die Sensoreinrichtung mit der Steuereinrichtung verbunden ist. Durch die Sensoreinrichtung können z.B. die Konzentration der Nährstoffe oder Stoffwechselprodukte in dem Nährmedium bestimmt werden. Durch die Kopplung der Sensoreinrichtung mit der Steuereinrichtung kann schließlich eine gegebenenfalls erforderliche Änderung der chemischen und physikalischen Zustandswerte des Nährmediums vorgenommen werden. Diese Änderung kann aufgrund der beschriebenen Kopplung nahezu in "Echtzeit" erfolgen, d.h. in unmittelbarer Folge auf die Ermittlung der entsprechenden Zustandswerte.

Schließlich ist der Bioreaktor in einer bevorzugten Ausgestaltung dadurch gekennzeichnet, daß ein geschlossenes Gehäuse vorgesehen ist, in welchem die Aufnahmeeinrichtung angeordnet ist und zumindest ein Zufluß und ein Abfluß für das Nährmedium sowie ein Zugang zum Einbringen und Abführen des organischen Materials vorgesehen sind. Durch das geschlossene Gehäuse ist gewährleistet, daß das Innere, insbesondere die Aufnahmeeinrichtung des Bioreaktors, nach dessen Herstellung und Sterilisierung auch nach Lagerung und Transport noch steril bleibt. Darüber hinaus wird eine kontaminationsfreie Kultivierung des organischen Materials ermöglicht, da die einzelnen Behandlungsschritte, z.B. das Einbringen und Abführen des organischen Materials sowie das Zu- und Abführen des Nährmediums oder anderer Substanzen, bei geschlossenem Gehäuse vorgenommen werden können und so die Gefahr einer Kontamination des organischen Materials stark vermindert werden kann. Durch den einfachen Aufbau des Bioreaktors ist dieser kostengünstig herstellbar und für einen Einsatz als Einmal-Artikel im klinischen Bereich besonders geeignet.

Ein Aspekt des erfindungsgemäßen Verfahrens liegt darin, das in Strömung versetzte Nährmedium durch die das organische Material haltende Aufnahmeeinrichtung hindurchzuleiten. Dadurch ist eine einfach handhabbare und zuverlässige Kultivierung des organischen Materials möglich. Insbesondere ist durch das Durchleiten des Nährmediums durch die Aufnahmeeinrichtung hindurch eine gute Versorgung des daran bzw. darin gehaltenen organischen Materials mit Nährstoffen gewährleistet, da ein beständiges Umspülen oder gegebenenfalls Durchsetzen mit Nährlösung erzielt wird. Die Strömung kann gleichbleibend oder gepulst sein.

Um die Kontaminationsgefahr für das zu kultivierende organische Material besonders gering zu halten, ist es vorteilhaft, wenn vor einem Beimpfen oder Einbringen des organischen Materials in die Aufnahmeeinrichtung diese sterilisiert wird. Die Sterilisation der Aufnahmeeinrichtung kann hierbei z.B. herstellerseitig unmittelbar nach der Herstellung des Bioreaktors vorgenommen werden. Durch geeignete Maßnahmen zum Verschließen des Bioreaktors kann die Sterilität der Aufnahmeeinrichtung dann bis zum Zeitpunkt des Einsatzes bewahrt werden. Es ist aber auch möglich, die Sterilisation der Aufnahmeeinrichtung erst unmittelbar vor dessen Einsatz vorzunehmen. Prinzipiell können aber zur Sicherung einer besonders hohen Kontaminationsfreiheit beide Maßnahmen kombiniert werden. Außerdem ist auch eine Sterilisation des gesamten Bioreaktors denkbar, wenn beispielsweise besonders hohe Anforderung an die Sicherung der Keimfreiheit gestellt werden.

Zur weiteren Vereinfachung des Kultivierungsprozesses ist vorgesehen, daß vor dem Abführen des kultivierten organischen Materials ein Medium, insbesondere eine physiologische Lösung mit einem Enzym, etwa eine Trypsinlösung zum Lösen und Ausspülen des angelagerten organischen Materials eingebracht wird. Hierdurch erübrigt sich ein Eingreifen in den geschlossenen Bioreaktor, wie es beispielsweise bei einem mechanischen Ablösen des angelagerten organischen Materials mit geeigneten Instrumenten erforderlich ist. Neben dieser Vereinfachung der Handhabung läßt sich durch dieses nichtinvasive Ablösen des Materials die Gefahr sowohl einer Kontamination als auch einer mechanischen Beschädigung des Gewebes stark vermindern. Die Spüllösung kann dabei durch denselben Zugang erfolgen, wie die Beimpfung mit organischem Material. Beim Beimpfen und Ausspülen, auch Ernten genannt, wird zweckmäßigerweise die Nährmediumströmung unterbrochen.

Eine vorteilhafte Ausgestaltung des Verfahrens sieht vor, daß die Strömungsrichtung des durch die Aufnahmeeinrichtung geleiteten Nährmediums während der Kultivierung des organischen Materials geändert wird. Hierdurch kann insbesondere bei organischem Material mit großer lateraler Ausdehnung und Dicke eine gute Versorgung der Zellen erreicht werden. Gegebenenfalls auftretende Nährstoffgradienten im Material können auf diese Weise deutlich reduziert werden.

Um eine an den jeweiligen zeitlichen Verlauf des Kultivierungsprozesses angepaßte Versorgung des organischen Materials mit Nährstoffen zu gewährleisten und damit eine intensive Kultivierung desselben zu erzielen, ist bei dem erfindungsgemäßen Verfahren vorgesehen, daß stoffliche Zusammensetzung, stöchiometrische Zusammensetzung, Zustand oder Durchflußgeschwindigkeit des Nährmediums während der Kultivierung geändert werden.

Hierbei ist außerdem von Vorteil, wenn während des Durchleitens des Nährmediums durch die Aufnahmeeinrichtung chemische und/oder physikalische Zustandswerte des Nährmediums gemessen werden, daß die gemessenen Zustandswerte ausgewertet werden und die chemischen und/oder physikalischen Zustandswerte des Nährmediums in Abhängigkeit von den gemessenen Zustandswerten geändert werden. Auf diese Weise lassen sich besonders gut chemische und/oder physikalische Parameter des Nährmediums oder zusätzlicher Nährstoffe an den sich ändernden Bedarf der Zellen im Verlauf des Kultivierungsprozesses anpassen. Es lassen sich auch insbesondere Stoffwechselprodukte des organischen Materials über einen Lactat- oder CO₂ -Wert im Nährmedium feststellen und zur Steuerung, Überwachung und Dokumentation des Kultivierungsprozesses sehr gut einsetzen. Es kann so etwa der beste Zeitpunkt zum Ernten ermittelt werden, beispielsweise unmittelbar vor einer unerwünschten Differenzierung bei der Kultivierung von Stammzellen.

Im folgenden wird die Erfindung anhand von Zeichnungen bevorzugter Ausführungsbeispiele näher beschrieben. Es zeigen in einer stark schematisierten Darstellung
- Fig. 1: einen Querschnitt einer bevorzugten Ausführungsform des erfindungsgemäßen Bioreaktors als Flachzelle;
- Fig. 2: eine Ansicht auf eine Zusammenstellung einzelner Komponenten eines erfindungsgemäßen Bioreaktors;
- Fig. 3: einen zusammengebauten Bioreaktor als Flachzelle; und
- Fig. 4: ein Diagramm einer Anlage mit einem Bioreaktor.

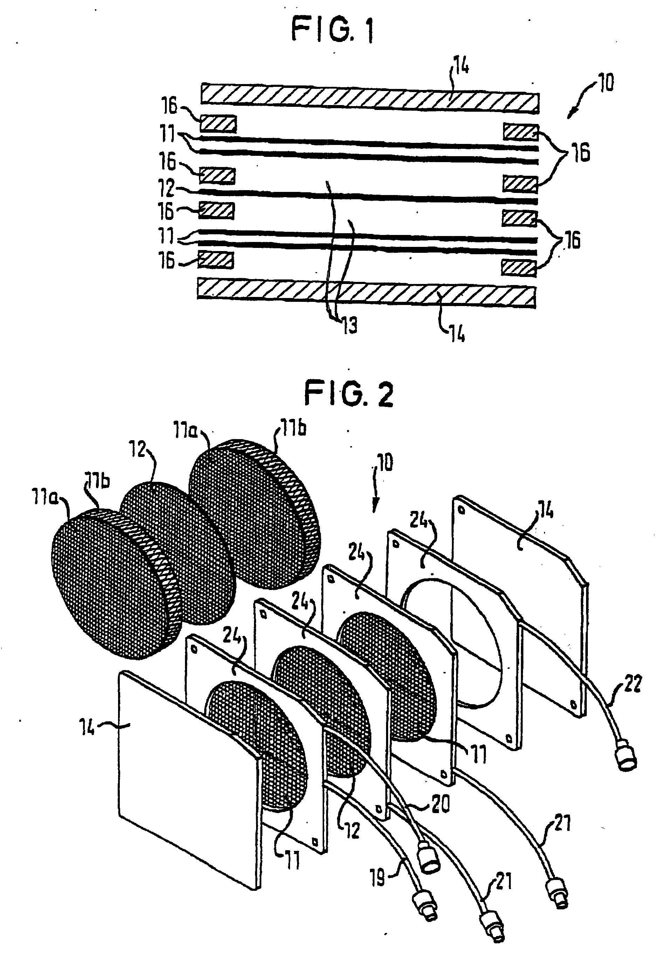

Der in Fig. 1 dargestellte Querschnitt einer bevorzugten Ausführungsform eines erfindungsgemäßen Bioreaktors 10 zur Kultivierung von Stammzellen weist ein Trägerelement 12, davon beabstandete, jeweils zweiteilige Trennwandelemente 11 sowie eine beidseitige Abdeckung 14 auf. Durch die zwischen den einzelnen Elementen vorgesehenen Abstandhalter 16, welche separate. Ringelemente oder Teil des Gehäuses sein können, ist eine Einstellung eines gewünschten Abstandes zwischen dem Trägerelement 12 und dem Trennwandelement 11 bzw. zwischen dem Trennwandelement 11 und der Abdeckung 14 möglich.

Die Abdeckungen 14 und die Abstandhalter 16 sind aus Polycarbonat hergestellt. Das Trägerelement 12 ist als technisches Gewebe ausgebildet. Als Fasermaterial wird vorzugsweise ein Monofilament aus Polyamid 6.6 (PA 6.6) oder Polyethylenterephtalat (PET) eingesetzt. Die Maschenweite des Gewebes beträgt durchschnittlich 20 µm und eine Dicke von etwa 55 bis 60 µm auf. Das Gewicht des Gewebes liegt bei etwa 35 bis 40 g/m².

Eine Aufnahmeeinrichtung für das organische Material ist durch das Trägerelement 12 und die Trennwandelemente 11 gebildet, welche einen Aufnahmeraum 13 der Aufnahmeeinrichtung seitlich begrenzen. In dem gezeigten Beispiel weisen die Trennwandelemente 12 jeweils eine Membran auf, welche auf einem darunterliegenden Stützgewebe aufgebracht ist. Das Membranmaterial umfaßt Polyamid 66 (PA 66). Als Stützgewebe dienen beispielsweise Monofilamentgewebe aus Polyethylenterephtalat (PET) mit einer Maschenweite von etwa 265 µm, einer Dicke von etwa 200 µm sowie einem Gewicht von typischerweise 85 g/m². Typische Membrandicken liegen zwischen 0,45 µm und 0,8 µm. Die Abstandhalter 16 weisen zur Beabstandung der einzelnen Elemente des Bioreaktors eine Höhe von etwa 3 mm auf.

Fig. 2 zeigt eine Ansicht auf eine Zusammenstellung einzelner Komponenten des erfindungsgemäßen Bioreaktors 10. Zwischen den beiden Abdeckungen 14 befinden sich mehrere ringförmige Trägerplatten 24, an welchen das Trägerelement 12 bzw. die Trennwandelemente 11 angebracht sind, welche aus einer Membran 11a und einem Stützgewebe 11b bestehen. Die kreisrunden Formen der verwendeten Gewebe bzw. Membranen sind paßgenau ausgeschnitten, beispielsweise mittels eines Lasers, wobei das als technische Gewebe ausgebildete Trägerelement 12 und die Trennwandelemente 11 auf den Trägerplatten 24 mittels unter UV-Licht aushärtbarem Klebstoff befestigt oder angeschweißt sind.

Die ringförmigen Trägerplatten 24 bilden mit den Abdeckungen 14 ein Gehäuse mit Hohlraum, in welchen Leitungen zum Zu- und/oder Abführen von Fluiden münden. Bei dem vorliegenden Ausführungsbeispiel erfolgt die Zuführung des Nährmediums in den Bioreaktor 10 über die Zuführleitung 19, während zum Abführen des Nährmediums die Abfuhrleitung 22 vorgesehen ist. Das Beimpfen des organischen Materials sowie das Zuführen eines enzymhaltigen Mediums zum Lösen des angelagerten organischen Materials erfolgt über zwei Leitungen 21, die in die Aufnahmeeinrichtung des Bioreaktors 10 münden. Zur Entlüftung des Bioreaktors 10 ist an der Trägerplatte 24 mit der Zuführleitung 19 eine Entlüftungsleitung 20 angeordnet.

Wie dieses Ausführungsbeispiel zeigt, übernehmen hier die im wesentlichen gleich aufgebauten Trägerplatten 24 die Funktion der in Fig. 1 dargestellten Abstandhalter 16. Durch Variation der Dicke der einzelnen Trägerplatten bzw. der Abstandshalter 16 kann der Abstand der einzelnen Bestandteile des Bioreaktors 10 an die für die Kultivierung bestimmter organischer Materialien erforderlichen Bedingungen hervorragend angepaßt werden. Eine wesentliche Rolle spielt hierbei das Verhältnis der Reaktoroberfläche zum Reaktorvolumen, welches durch die Wahl der entsprechenden Gewebe einstellbar ist. Im übrigen läßt sich der Abstand zwischen den einzelnen Bestandteilen auch beispielsweise durch das Einlegen unterschiedlicher Dichtungen oder Zwischenplatten leicht verändern, wobei durch lösbare Befestigungsmittel auch eine Demontage möglich ist.

An die Abdeckungen 14 können sich außerdem Temperierkammern (nicht dargestellt) anschließen, welche zur Aufrechterhaltung einer gleichmäßigen Prozeßtemperatur bei der Kultivierung des organischen Materials dienen. Typischerweise verfügen solche Temperierkammern über eine elektrische Heizung und/oder Kühlung oder sind über Zu- und Ableitungen an einem Wärme- oder Kühlbad angeschlossen. Prinzipiell ist aber auch die Lagerung des Bioreaktors in einem temperierten Wärmeschrank möglich.

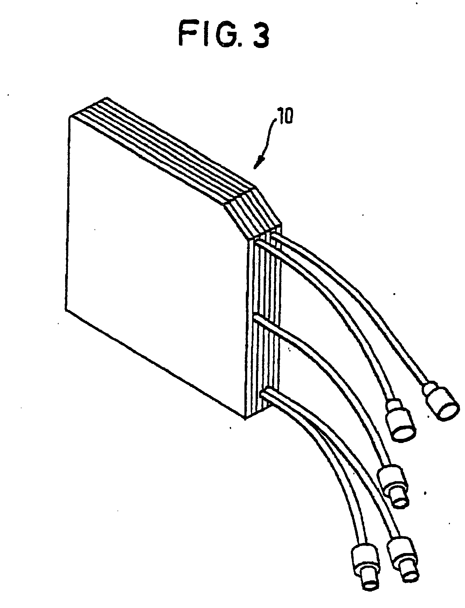

Der in Fig. 2 mit seinen einzelnen Bestandteilen beschriebene Bioreaktor 10 ist in der Fig. 3 als zusammengesetzte, fertige Flachzelle mit Zuführ- und Abführleitungen dargestellt. Der erfindungsgemäße Bioreaktor 10 besitzt ein kompaktes Format, welches dessen Handhabung im Laborbereich ebenso wie im klinischen Bereich besonders erleichtert.

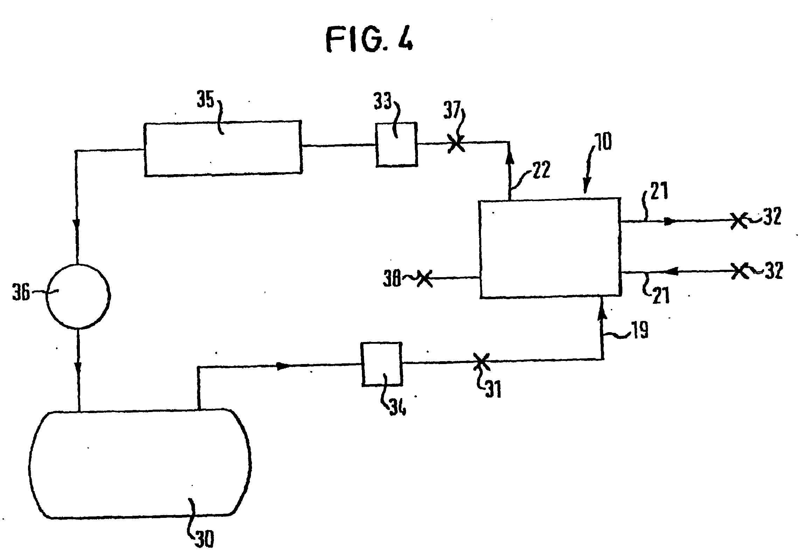

Im Zusammenhang mit dem Schaubild gemäß Fig. 4 werden kurz die wichtigsten Schritte beim Betrieb des Bioreaktors 10 erläutert, welcher zur Kultivierung von peripheren, hämatopoetischen Stammzellen eingesetzt wird. Diese Stammzellen werden zur Blutstammzellentransplantation nach einer Hochdosischemotherapie benötigt.

Zum Beimpfen des Bioreaktors 10 wird dieser zunächst über eine Zuführleitung 19 aus einem Behälter 30 mit Nährmedium gefüllt, welches in radialer Richtung in den Reaktorhohlraum einströmt. Die entsprechende Zuführleitung 19 wird zunächst über ein Sperrventil 31 geschlossen, während eine Abführleitung 22 offen bleibt. Die für die Beimpfung vorgesehenen Zuführleitungen 21 werden über ihre Zuleitungs-Sperrventile 32 geöffnet und die Stammzellen, welche einem Patienten vor der Strahlen- oder Chemotherapie entnommen wurden, können durch diese beispielsweise mit einer Spritze in das Reaktormodul 10 eingespült werden. Nach diesem Vorgang werden die für die Beimpfung vorgesehenen Zuführleitungen 21 sowie die Abführleitung 22 geschlossen. Die Stammzellen befinden sich in der Aufnahmeeinrichtung und können diese besiedeln.

Nach einer gewissen Zeit, welche für eine gleichmäßige Verteilung und Besiedelung der Stammzellen vorteilhaft ist, werden zum Fördern des Zellwachstums die Zuführleitung 19 sowie eine Abführleitung 22 geöffnet. Durch die Zuführleitung 19 wird dann temperierte Nährlösung zugeführt. Im Verlauf des Kultivierungsprozesses kann die Menge der pro Zeiteinheit zugeführten Nährlösung allmählich erhöht werden, da mit zunehmender Dauer des Kultivierungsprozesses mehr Zellen vorhanden sind und somit der Nährstoffbedarf anwächst.

Die Zu- und Ableitung des Nährmediums erfolgt so, daß der im Querschnitt kreisförmige Hohlraum des Bioreaktors 10 und damit die Aufnahmeeinrichtung möglichst gleichmäßig durchströmt wird. Hierdurch wird über die gesamte Fläche des Zellträgers ein gleicher und gleichbleibender Nährstoffgradient sichergestellt. Gleichzeitig werden schädliche Stoffwechselprodukte durch die Nährstoffströmung schnell und zuverlässig aus dem Bioreaktor 10 abgeführt.

Nach Austritt des Nährmediums über die Abführleitung 22 durchläuft das Nährmedium eine erste Meßeinrichtung 33, mit welcher der Gehalt an Nährstoffen und Schadstoffen sowie weitere physikalische und chemische Zustandswerte erfaßt werden. Die ermittelten Werte dienen zur Steuerung und Überwachung des Kultivierungsprozesses mittels einer entsprechenden Steuereinrichtung. Diese kann weiter mit einer zweiten Meßeinrichtung 34 in der Zuführleitung 19 verbunden sein. Das Nährmedium kann nach einer durchgeführten Reinigung und Wiederaufbereitung in einer Aufbereitungseinheit 35 im Kreislauf in den Behälter 30 rückgeführt werden, wobei eine Pumpe 36 als Strömungserzeugungseinrichtung vorgesehen ist.

Zur Entnahme kultivierter Zellen werden schließlich über die Leitung 21 Enzyme zugeführt, welche eine Ablösung der Stammzellen vom Trägerelement 12 bewirken. Die Abführleitung 22 wird dabei über ein Sperrventil 37 geschlossen und eine physiologische Lösung wird zugeführt. Die Stammzellen können nun mittels einer Spritze durch die für die Beimpfung vorgesehene Leitung 21 abgesaugt werden. Die hierdurch gewonnenen Stammzellen können nun gewaschen und gegebenenfalls weiteren Behandlungsschritten unterzogen werden, bevor sie einer weiteren Verwendung, z.B. als Blutersatzstoff oder Implantat, zugeführt werden. Zur Entlüftung ist an dem Modulgehäuse des Bioreaktors 10 ein Entlüftungsventil 38 angeordnet.

Aus den Fig. 8 und 9 ist ein weiterer erfindungsgemäßer Bioreaktor 10c zu entnehmen. Innerhalb einer zylindrischen Außenwand 8c ist wie bei den vorbeschriebenen Ausführungsformen koaxial zueinander ein Trennwandelement 11c, ein Trägerelement 12c sowie ein innenliegendes Trennwandelement 11c vorgesehen. Zur Formstabilisierung ist das äußere Trennwandelement 11c an einem ringförmigen Stützkörper 15c angebracht, wobei mehrere ringförmige Stützkörper axial über eingebettete Distanzhalter 19c miteinander verbunden sind. An der radialen Innenseite des ringförmigen Stützkörpers 15c ragen in einem Winkelabstand von 90^{°} zueinander Radialstege 23 nach innen, welche an dem rohrförmigen Trägerelement 12c enden. Innerhalb des rohrförmigen Trägerelementes 12c ist wiederum ein kreuzförmiges Stützelement 21c mit einer Mittenöffnung vorgesehen, in welchem koaxial das Innere zylindrische Trennwandelement 11c angeordnet ist.

Ein mit dem vorbeschriebenen Bioreaktor 10c vergleichbarer Bioreaktor 10d ist in Fig. 10 gezeigt. Allerdings sind bei dem Bioreaktor 10d insgesamt drei innere Trennwandelemente 11d vorgesehen, welche in einem Winkelabstand von 120° zueinander innerhalb des zylindrischen Trägerelementes 12d angeordnet sind.

Bei dem rohrartig aufgebauten Bioreaktor 11e gemäß Fig. 11 ist das Trägerelement 12e plissiert, d.h. wellenförmig zwischen den beiden koaxial zueinander angeordneten Trennwandelemente 11e ausgebildet. Hierdurch wird eine besonders große Oberfläche des Trägerelementes 12e erreicht.

Bei einem anderen Bioreaktor 10f gemäß Fig. 12, welcher ähnlich dem Bioreaktor 10c gemäß Fig. 8 und 9 ausgebildet ist, ist das Trägerelement 12f netz- oder gerüstartig zwischen den zwei Trennwandelementen 11f angeordnet, welche durch einen Stützkörper 15f gehalten werden.

Ein vergleichbar zu dem Bioreaktor 10d von Fig. 10 ausgebildeter anderer Bioreaktor 10g geht aus Fig. 13 hervor. Hier ist ebenfalls das Trägerelement 10g netz- oder gerüstartig in dem Raum zwischen den zwei Trennwandelementen 10g angeordnet.

Ein weiterer erfindungsgemäßer Bioreaktor 10h ist in Fig. 14 dargestellt. Eine zylindrische Außenwand 8h umfasst ein Stützgerüst bestehend aus einem ringförmigen Stützkörper 15h und einem darin über vier Radialstege 23h gehaltenen, etwa kleeblattförmigen inneren Stützkörper 21h. An der Außenseite des ringförmigen Stützkörpers 15h ist das äußere Trennwandelement 11h angebracht, während in dem inneren Stützkörper 21h insgesamt neun rohrförmige Trennwandelemente 11h angeordnet sind. Zur Vergrößerung der Oberfläche des Trägerelementes 12h ist dieses plissiert, d.h. mit einer gewissen Wellenform ausgebildet.

## Patentansprüche

1. Bioreaktor zur Kultivierung organischen Materials, insbesondere von Zellen, mittels eines Nährmediums, welches in eine Strömung versetzbar ist, mit
- einem Gehäuse,
- einer darin angeordneten Aufnahmeeinrichtung, welche einen von dem Nährmedium durchströmbaren Aufnahmeraum (13) für das organische Material aufweist,
- mindestens zwei Trennwandelementen (11), welche den Aufnahmeraum (13) umschließen und j eweils eine Membran (11a) aufweisen, die einerseits für das Nährmedium durchströmbar und andererseits im Wesentlichen undurchlässig für das organische Material ist, und
- einem im Aufnahmeraum (13) angeordneten, für das Nährmedium durchlässigen Trägerelement (12), welches als ein Gewebe zum Anlagern des organischen Materials ausgebildet ist,
**dadurch gekennzeichnet,**
- **dass** das Gehäuse als eine Flachzelle mit ringförmigen Trägerplatten (24) aufgebaut ist,
- **dass** die Trennwandelemente (11) ein Stützgewebe (11b) aufweisen, auf welchem die Membran (11a) aufgebracht ist und
- **dass** sowohl das Stützgewebe (11b) mit der aufgebrachten Membran (11a) als auch das Gewebe des Trägerelementes (12) jeweils in einer ringförmigen Trägerplatte (24) befestigt sind.

2. Bioreaktor nach Anspruch 1,
**dadurch gekennzeichnet,**
**daß** das Trägerelement (12) eine dreidimensionale Struktur aufweist, insbesondere als dreidimensionales Gewebe ausgebildet ist.

3. Bioreaktor nach Anspruch 1 oder 2,
**dadurch gekennzeichnet,**
**daß** das Trägerelement (12) ein textiles Trägermaterial umfaßt.

4. Bioreaktor nach Anspruch 3,
**dadurch gekennzeichnet,**
- **daß** das textile Trägermaterial oberflächenbehandelt ist und
- **daß** eine bioverträgliche Oberfläche mit einer Struktur für eine Adhäsion des organischen Materials ausgebildet ist.

5. Bioreaktor nach einem der Ansprüche 1 bis 4,
**dadurch gekennzeichnet,**
**daß** die Aufnahmeeinrichtung der Flachzelle (9) kreisrund ausgebildet ist.

6. Bioreaktor nach einem der Ansprüche 1 bis 5,
**dadurch gekennzeichnet,**
**daß** mehrere Flachzellen (9) als Module in einer Strömungsrichtung parallel und/oder seriell angeordnet sind.

7. Bioreaktor nach einem der Ansprüche 1 bis 6,
**dadurch gekennzeichnet,**
**daß** eine Steuereinrichtung vorgesehen ist, durch welche eine Strömungserzeugungs-Einrichtung, eine. Temperatureinstelleinheit, eine Begasungseinheit, eine Entgasungseinheit und/oder weitere Versorgungseinheiten steuerbar und/oder regelbar sind.

8. Bioreaktor nach Anspruch 7,
**dadurch gekennzeichnet,**
- **daß** in einer Strömungsrichtung nach dem Aufnahmeraum (13) eine Sensoreinrichtung angeordnet ist, durch welche physikalische und chemische Zustandswerte des Nährmediums ermittelbar sind und
- **daß** die Sensoreinrichtung mit der Steuereinrichtung verbunden ist.

9. Bioreaktor nach einem der Ansprüche 1 bis 8,
**dadurch gekennzeichnet**, demontierbares
- daß ein geschlossenes, insbesondere demontierbares - Gehäuse vorgesehen ist, in welchem die Aufnahmeeinrichtung angeordnet ist, und
- daß zumindest ein Zufluß und ein Abfluß für das Nährmedium sowie ein Zugang zum Einbringen und Abführen des organischen Materials vorgesehen sind.

10. Verfahren zur Kultivierung organischen Materials,
bei dem
- ein Nährmedium zumindest zeitweise in eine Strömung versetzt wird,
- das organische Material in eine Aufnahmeeinrichtung eines Bioreaktors (11) eingebracht wird und
- das Nährmedium durch die Aufnahmeeinrichtung des Bioreaktors (11) für eine'konvektive Versorgung des organischen Materials durchgeleitet wird,
**dadurch gekennzeichnet,**
- **daß** ein Bioreaktor (11) nach einem der Ansprüche 1 bis 9 verwendet wird.

11. Verfahren nach Anspruch 10,
**dadurch gekennzeichnet,**
**daß** vor einem Beimpfen oder einem Einbringen des organischen Materials in die Aufnahmeeinrichtung diese sterilisiert wird.

12. Verfahren nach Anspruch 10 oder 11,
**dadurch gekennzeichnet,**
**daß** vor einem Abführen des kultivierten organischen Materials aus der Aufnahmeeinrichtung ein Medium, insbesondere ein Enzym, zum Lösen von angelagertem organischen Material eingebracht wird.

13. Verfahren nach einem der Ansprüche 10 bis 12,
**dadurch gekennzeichnet,**
**daß** die Strömungsrichtung des durch die Aufnahmeeinrichtung geleiteten Nährmediums während der Kultivierung des organischen Materials geändert wird.

14. Verfahren nach einem der Ansprüche 10 bis 13,
**dadurch gekennzeichnet,**
**daß** ein chemischer und/oder physikalischer Zustand des Nährmediums, insbesondere eine stoffliche Zusammensetzung, eine stöchiometrische Zusammensetzung, Temperatur, Druck oder Durchflußgeschwindigkeit, im zeitlichen Verlauf der Kultivierung gezielt verändert werden.

15. Verfahren nach einem der Ansprüche 10 bis 13,
**dadurch gekennzeichnet,**
- **daß** zumindest nach dem Durchleiten des Nährmediums durch die Aufnahmeeinrichtung chemische und/oder physikalische Zustandswerte des Nährmediums gemessen werden,
- **daß** die gemessenen Zustandswerte in einer Steuereinrichtung erfaßt und ausgewertet werden, und
- **daß** die gemessenen Zustandswerte zur Steuerung und/oder Regelung des Verlaufes der Kultivierung. des organischen Materials eingesetzt werden.

16. Verfahren nach einem der Ansprüche 10 bis 15,
**dadurch gekennzeichnet,**
- **daß** das Nährmedium durch mehrere Aufnahmeeinrichtungen geleitet wird, welche parallel und/oder seriell zueinander angeordnet sind.

## Claims

1. Bioreactor for cultivating organic material, in particular cells, by means of a nutrient medium, which can be put into a flow, comprising
- a housing,
- a receiving device arranged therein, which has a receiving space (13) for the organic material that can be flowed through by the nutrient medium,
- at least two partition wall elements (11), which enclose the receiving space (13) and each have a membrane (11a), which is on the one hand permeable to the nutrient medium and on the other hand substantially impermeable to the organic material, and
- a carrier element (12) arranged in the receiving space (13), which is permeable to the nutrient medium and is designed as a fabric for an adhesion of the organic material,
**characterized in that**
- the housing is constructed as a flat cell having annular carrier plates (24),
- the partition wall elements (11) have a supporting fabric (11b), to which the membrane (11a) is applied and
- both the supporting fabric (11b) with the applied membrane (11a) and the fabric of the carrier element (12) are each mounted in an annular carrier plate (24).

2. Bioreactor according to claim 1,
**characterized in that**
the carrier element (12) has a three-dimensional structure, in particular it is designed as three-dimensional fabric.

3. Bioreactor according to claim 1 or 2,
**characterized in that**
the carrier element (12) includes a textile carrier material.

4. Bioreactor according to claim 3,
**characterized in that**
- the textile carrier material is surface-treated and
- a bio-compatible surface is formed with a structure adapted for an adhesion of the organic material.

5. Bioreactor according to any one of claims 1 to 4,
**characterized in that**
the receiving device of the flat cell (9) is designed circularly.

6. Bioreactor according to any one of claims 1 to 5,
**characterized in that**
a number of flat cells (9) are arranged as modules in one flow direction in a parallel and/or serial fashion.

7. Bioreactor according to any one of claims 1 to 6,
**characterized in that**
a control device is provided, by means of which a flow generating device, a temperature adjusting unit, a gasing unit, a degasing unit and/or further supply units can be controlled and/or regulated.

8. Bioreactor according to claim 7,
**characterized in that**
- a sensor device is arranged in a flow direction after the receiving space (13), by means of which physical and chemical values of state of the nutrient medium can be determined and
- the sensor device is connected to the control device.

9. Bioreactor according to any one of claims 1 to 8,
**characterized in that**
- a closed, in particular dismountable housing is provided, in which the receiving device is arranged, and
- at least one feed and one discharge are provided for the nutrient medium as well as an access for introducing and removing the organic material.

10. Method for cultivating organic material, wherein
- a nutrient medium is at least temporarily put into a flow,
- the organic material is introduced into a receiving device of a bioreactor (11) and
- the nutrient medium is passed through the receiving device of the bioreactor (11) for a convective supply of the organic material,
**characterized in that**
- a bioreactor (11) according to any one of claims 1 to 9 is used.

11. Method according to claim 10,
**characterized in that**
prior to an inoculation or introduction of the organic material into the receiving device this is sterilized.

12. Method according to claim 10 or 11,
**characterized in that**
prior to a removal of the cultivated organic material from the receiving device a medium, in particular an enzyme, is introduced for detaching adhered organic material.

13. Method according to any one of claims 10 to 12,
**characterized in that**
the direction of flow of the nutrient medium that is passed through the receiving device is changed during the cultivation of the organic material.

14. Method according to any one of claims 10 to 13,
**characterized in that**
a chemical and/or physical state of the nutrient medium, in particular a material composition, a stoichiometrical composition, temperature, pressure or rate of flow, are specifically changed in the course of time of the cultivation.

15. Method according to any one of claims 10 to 13,
**characterized in that**
- at least after passing the nutrient medium through the receiving device chemical and/or physical values of state of the nutrient medium are measured,
- the measured values of state are recorded and analysed in a control device, and
- the measured values of state are employed for controlling and/or regulating the course of the cultivation of the organic material.

16. Method according to any one of claims 10 to 15
**characterized in that**
- the nutrient medium is passed through a number of receiving devices, which are arranged to each other in a parallel and/or serial fashion.

## Revendications

1. Bioréacteur pour la culture de matières organiques, en particulier de cellules, au moyen d'un milieu nutritif qui peut être mis en écoulement, avec
- un boîtier,
- un dispositif récepteur à l'intérieur dans celui-ci, qui présente un logement (13) pour la matière organique pouvant être traversé par le milieu nutritif,
- au moins deux éléments de cloison (11) qui entourent le logement (13) et présentent chacun une membrane (11a), qui peut d'une part être traversée par le milieu nutritif et est d'autre part sensiblement imperméable à la matière organique, et
- un élément support (12) placé dans le logement (13) et perméable au milieu nutritif, qui est conformé en tissu pour accumuler la matière organique,
***caractérisé***
***en ce que*** le boîtier est construit comme une cellule plate avec des plaques supports annulaires (24),
***en ce que*** les éléments de cloison (11) présentent un tissu porteur (11b) sur lequel est appliquée la membrane (11a), et
***en ce que*** à la fois le tissu porteur (11b) avec la membrane rapportée (11a) et le tissu de l'élément support (12) sont fixés chacun dans une plaque support annulaire (24).

2. Bioréacteur selon la revendication 1, **caractérisé en ce que** l'élément support (12) présente une structure tridimensionnelle, en particulier est conformé en tissu tridimensionnel.

3. Bioréacteur selon la revendication 1 ou 2, ***caractérisé* en ce que** l'élément support (12) comprend un matériau support textile.

4. Bioréacteur selon la revendication 3, ***caractérisé***
***en ce* que** le matériau support textile est traité en surface, et
**en *ce qu***'une surface biocompatible est réalisée, avec une structure pour une adhésion de la matière organique.

5. Bioréacteur selon l'une quelconque des revendications 1 à 4, ***caractérisé en ce que*** le dispositif récepteur de la cellule plate (9) est réalisé circulaire.

6. Bioréacteur selon l'une quelconque des revendications 1 à 5, ***caractérisé en ce que*** plusieurs cellules plates (9) sont placées en modules en parallèle et/ou en série dans une direction d'écoulement.

7. Bioréacteur selon l'une quelconque des revendications 1 à 6, ***caractérisé en ce qu***'il est prévu un dispositif de commande grâce auquel un dispositif de production d'écoulement, une unité de réglage de température, une unité d'alimentation en gaz, une unité d'évacuation de gaz et/ou d'autres unités d'alimentation peuvent être pilotées et/ou régulées.

8. Bioréacteur selon la revendication 7, ***caractérisé***
***en ce qu***'après le logement (13) dans une direction d'écoulement, il est placé un dispositif à capteur grâce auquel des valeurs d'état physiques et chimiques du milieu nutritif peuvent être déterminées, et
***en ce que*** le dispositif à capteur est relié au dispositif de commande.

9. Bioréacteur selon l'une quelconque des revendications 1 à 8, ***caractérisé***
***en* ce *qu***'il est prévu un boîtier fermé, en particulier démontable, dans lequel est placé le dispositif de réception, et
***en* ce *qu***'au moins une arrivée et un départ de milieu nutritif sont prévus, ainsi qu'un accès pour introduire et retirer la matière organique.

10. Procédé de culture de matière organique, dans lequel :
un milieu nutritif est au moins temporairement mis en écoulement,
la matière organique est introduite dans un dispositif récepteur d'un bioréacteur (11), et
le milieu nutritif est envoyé à travers le dispositif récepteur du bioréacteur (11) pour une alimentation convective de la matière organique,
***caractérisé en ce que*** l'on utilise un bioréacteur selon l'une quelconque des revendications 1 à 9.

11. Procédé selon la revendication 10, ***caractérisé en ce qu***'avant une inoculation ou une introduction de la matière organique dans le dispositif récepteur, celui-ci est stérilisé.

12. Procédé selon la revendication 10 ou 11,
***caractérisé en ce qu***'avant d'extraire la matière organique cultivée du dispositif récepteur, un milieu, en particulier un enzyme, est introduit pour détacher la matière organique déposée.

13. Procédé selon l'une quelconque des revendications 10 à 12, ***caractérisé en ce que*** la direction d'écoulement du milieu nutritif envoyé à travers le dispositif récepteur est modifiée pendant la culture de la matière organique.

14. Procédé selon l'une quelconque des revendications 10 à 13, ***caractérisé en ce qu***'un état chimique et/ou physique du milieu nutritif, en particulier une composition de matière, une composition stoechiométrique, la température, la pression ou la vitesse d'écoulement, est modifié de manière cible au cours de la culture.

15. Procédé selon l'une quelconque des revendications 10 à 13, ***caractérisé***
***en ce qu***'au moins après l'envoi du milieu nutritif à travers le dispositif récepteur, des valeurs d'état chimiques et/ou physiques du milieu nutritif sont mesurées,
***en ce que*** les valeurs d'état mesurées sont relevées et analysées dans un dispositif de commande, et
***en ce que*** les valeurs d'état mesurées sont utilisées pour commander et/ou réguler le déroulement de la culture de la matière organique.

16. Procédé selon l'une quelconque des revendications 10 à 15, ***caractérisé en ce que*** le milieu nutritif est envoyé à travers plusieurs dispositifs récepteurs, qui sont disposés en parallèle et/ou en série.
